(19) Europäisches Patentamt
European Patent Office
Office européen des brevets

(11) EP 4 394 375 B1

(12) **EUROPEAN PATENT SPECIFICATION**

(45) Date of publication and mention of the grant of the patent:
**22.07.2026 Bulletin 2026/30**

(21) Application number: **21954971.4**

(22) Date of filing: **24.08.2021**

(51) International Patent Classification (IPC):
***G01N 33/00*** *(2006.01)* ***G01N 27/62*** *(2021.01)*
***G01N 30/88*** *(2006.01)* *G01N 30/54 (2006.01)*
*G01N 30/72 (2006.01)*

(52) Cooperative Patent Classification (CPC):
**G01N 27/62; G01N 30/88;** G01N 30/54;
G01N 30/72

(86) International application number:
**PCT/JP2021/030949**

(87) International publication number:
**WO 2023/026354 (02.03.2023 Gazette 2023/09)**

(54) **METHOD AND DEVICE FOR SCREENING SAMPLE CONTAINING PHENOL, ISOPROPYLATED PHOSPHATE (3:1)**

VERFAHREN UND VORRICHTUNG ZUM SCREENING EINER PHENOL, ISOPROPYLIERTES PHOSPHAT (3:1), ENTHALTENDEN PROBE

PROCÉDÉ ET DISPOSITIF DE CRIBLAGE D'UN ÉCHANTILLON CONTENANT DU PHÉNOL, PHOSPHATE ISOPROPYLÉ (3:1)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.07.2024 Bulletin 2024/27**

(73) Proprietor: **Shimadzu Corporation**
**Nakagyo-ku,**
**Kyoto-shi,**
**Kyoto 604-8511 (JP)**

(72) Inventors:
- **KUDO, Yukihiko**
  **Kyoto-shi, Kyoto 604-8511 (JP)**
- **OBAYASHI, Kenichi**
  **Kyoto-shi, Kyoto 604-8511 (JP)**
- **ISHII, Toshinari**
  **Kyoto-shi, Kyoto 604-8511 (JP)**
- **CHU, Xue**
  **Kyoto-shi, Kyoto 604-8511 (JP)**
- **KONDO, Tomoaki**
  **Kyoto-shi, Kyoto 604-8511 (JP)**
- **NAKAGAWA, Katsuhiro**
  **Kyoto-shi, Kyoto 604-8511 (JP)**

(74) Representative: **Müller-Boré & Partner**
**Patentanwälte PartG mbB**
**Friedenheimer Brücke 21**
**80639 München (DE)**

(56) References cited:
**CN-A- 113 219 091 US-A1- 2012 101 015**

- **ROSENBERGER W ET AL: "Triphenyl phosphate, isopropylated (isopropylated phenyl phosphates, IPPhP) -Determination of isopropylated phenyl phosphates in the workplace air using gas chromatography (GC-MS) Air Monitoring Method -Translation of the German version from 2020", vol. 6, no. 1, 31 March 2021 (2021-03-31), XP093206950, Retrieved from the Internet <URL:https://www.zora.uzh.ch/id/eprint/215440/1/am6893741e6_1or.pdf> [retrieved on 20240924], DOI: 10.34865/am6893741e6_1or**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 394 375 B1

- ISHII TOSHINARI, ET AL.: "Analysis of PIP (3:1) in resin using pyrolysis (thermal desorption) -GC-MS", SHIMADZU APPLICATION NEWS, 18 June 2021 (2021-06-18), XP093038572, Retrieved from the Internet <URL:https://www.an.shimadzu.co.jp/sites/an.shimadzu.co.jp/files/pim/pim_document_file/an_jp/applications/application_note/17392/an_01-00191-jp.pdf> [retrieved on 20230412]
- DU BIBAI; ZHANG YUN; CHEN HUI; SHEN MINGJIE; ZHOU WEI; ZENG LIXI: "Development and validation of a liquid chromatography-tandem mass spectrometry method for the simultaneous determination of 17 traditional and emerging aryl organophosphate esters in indoor dust", JOURNAL OF CHROMATOGRAPHY A, ELSEVIER, AMSTERDAM, NL, vol. 1603, 24 June 2019 (2019-06-24), AMSTERDAM, NL, pages 199 - 207, XP085788804, ISSN: 0021-9673, DOI: 10.1016/j.chroma.2019.06.045
- KŘIŽ J., ADAMCOVÁ E., TŘSKA J., VODICKA L.: "Capillary gas chromatography of isopropyl phenyl phosphates on quartz columns", JOURNAL OF HIGH RESOLUTION CHROMATOGRAPHY., WILEY VCH, WEINHEIM., DE, vol. 8, no. 4, 1 April 1985 (1985-04-01), DE , pages 195 - 197, XP093038574, ISSN: 0935-6304, DOI: 10.1002/jhrc.1240080410

**Description**

TECHNICAL FIELD

The present invention relates to a method and a device for screening a sample containing phenol, isopropylated phosphate.

BACKGROUND ART

Phenol, isopropylated phosphate (3:1) (PIP (3:1), CAS: 68937-41-7) is widely used as additives for imparting flame retardancy and plasticity to resin products such as poly vinyl chloride (PVC). In addition, PIP (3:1) also has abrasion resistance and pressure resistance, and thus is widely used as an additive for a hydraulic fluid, an adhesive, and the like. In Non Patent Literature 1 issued by the Environmental Protection Agency (EPA) of the United States, PIP (3:1) is defined as a group of compounds having a molecular structure in which triphenyl phosphate is a basic structure and all three phenyl groups have one or more isopropyl groups as substituents.

The EPA has certified PIP (3:1) as one of the substances having persistent, bioaccumulative, and toxic properties (PBT), based on the Toxic Substances Control Act (TSCA) of the United States (Non Patent Literature 2). Accordingly, export of products containing PIP (3:1) to the United States is restricted. In order to comply with this regulation, it is necessary to confirm previously that products to be exported to the United States do not contain PIP (3:1).

Non Patent Literatures 3 and 4 state that a sample solution in which a sample is dissolved is analyzed using LC/MS or GC/MS, and the amount of PIP (3:1) contained in the sample can be measured. In addition, Non Patent Literature 5 states that PIP (3:1) contained in the sample can be measured by using Py/TD-GC/MS which is a combination of Py/TD and the gas chromatography mass spectrometry, where Py/TD is a method of thermally desorbing (TD) components such as PIP (3:1) contained in a sample by a pyrolyzer (Py) which heats the sample as it is. Non Patent Literature 6 describes the determination of isopropylated phenyl phosphates in the workplace air using gas chromatography (GC-MS) air monitoring method.

CITATION LIST

NON PATENT LITERATURE

Non Patent Literature 1: Office of Chemical Safety and Pollution Prevention U.S. EPA, "Preliminary Information on Manufacturing, Processing, Distribution, Use, and Disposal: Phenol, isopropylated, phosphate (3:1)", Support document for Docket EPA-HQ-OPPT-2016-073 (2017)

Non Patent Literature 2: News Releases from Headquarters, "EPA Finalizes Action Protecting Americans from PBT Chemicals", United States Environmental Protection Agency, December 22, 2020, [Searched on August 20, 2021], Internet < URL: https://www.epa.gov/newsreleases/epa-finalizes-action-protecting-americans-pbt-chemicals>

Non Patent Literature 3: Martin Schlummera et al., "Analysis of flame retardant additives in polymer fractions of waste of electric and electronic equipment (WEEE) by means of HPLC-UV/MS and GPC-HPLC-UV", Journal of Chromatography A, Volume 1064, Issue 1, 28 January 2005, Pages 39-51

Non Patent Literature 4: Thomas Roth et al., "Gas chromatographic determination of phosphate-based flame retardants in styrene-based polymers from waste electrical and electronic equipment", Volume 1262, 2 November 2012, Pages 188-195

Non Patent Literature 5: Toshinari ISHII, Yukihiko KUDO, Kenichi OBAYASHI, "Application news pyrolysis (thermal desorption) - Analysis of PIP (3:1) in resin using GC/MS", [online], June 2021, Shimadzu Corporation, [searched on August 13, 2021], Internet <URL: https://www.an.shimadzu.co.jp/aplnotes/gcms/an_01-00191-jp.pdf>

Non Patent Literature 6: Rosenberger W et al., "Triphenyl phosphate, isopropylated (isopropylated phenyl phosphates, IPPhP) -Determination of isopropylated phenyl phosphates in the workplace air using gas chromatography (GC-MS) Air Monitoring Method -Translation of the German version from 2020", THE MAK COLLECTION FOR OCCUPATIONAL HEALTH AND SAFETY 2021, vol. 6, no. 1, March 2021

SUMMARY OF INVENTION

TECHNICAL PROBLEM

PIP (3:1) includes a large number of compounds having different numbers of substitutions or different substitution positions of the isopropyl group of the phenyl group, but when mass spectrometry is used as in Non Patent Literatures 3 to 5, a group of compounds having the same number of substitutions of the isopropyl group are detected as ions having one

common mass-to-charge ratio, whereby the group of compounds can be measured at a time. However, since the composition ratio of compound groups with each substitution number differs due to differences in manufacturing conditions and other factors, it may be difficult to detect a compound group of a substitution number with low composition ratio. Even if the constituent ratio is the same, because the measurement sensitivity of compound groups having each number of substitutions is also different, a compound group of a substitution number having considerable composition ratio may be sometimes difficult to detect. Therefore, it is difficult to determine that the sample contains PIP (3:1) based on the detection of all the compound groups corresponding to PIP (3:1).

In order to simplify the measurement, it is possible to measure only a part of all the compound groups having different numbers of substituents. However, as described above, the composition ratio (concentration) of each compound group in the product varies depending on the production conditions, and the measurement sensitivity of each compound group also varies, and thus it is difficult to determine which compound group should be set as the measurement target when screening a sample of a product or the like containing PIP (3:1). For example, when the content of the compound group to be measured is low or the measurement sensitivity is low, it was possible to miss the presence of PIP (3:1) in the sample. In addition, in a case where only one type of the compound group is to be measured, when ions of a foreign substance having the same mass-to-charge ratio as that of the compound group are present, it was possible to misidentify the content of the foreign substance as the content of the compound group and erroneously determine the presence of PIP (3:1) even though it is not actually contained.

An object of the present invention is to provide a screening technique capable of easily and correctly determining whether a sample to be analyzed contains phenol, isopropylated phosphate (3:1).

SOLUTION TO PROBLEM

A method for screening a sample containing phenol, isopropylated phosphate (3:1) according to the present invention made in order to solve the above problems includes:

a step of acquiring a content of a compound group represented by the following chemical formula 1 (x, y, and z are the number of isopropyl groups each phenyl group has as a substituent) in a sample, or a value corresponding to the content as a measured value, for each of a plurality of compound groups among a compound group in which a total of x, y, and z that three phenyl groups have is 1, a compound group in which the total is 2, and a compound group in which the total is 3; and

[Chemical Formula 1]

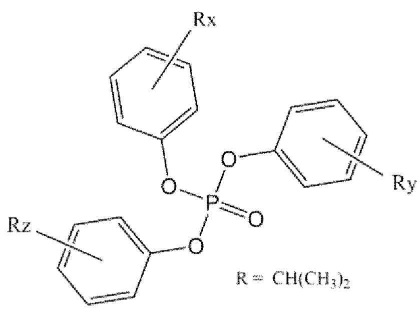

a step of determining that the sample contains phenol, isopropylated phosphate (3:1) based on a result of comparing the measured value with a threshold determined previously for the compound group corresponding to the measured value.

A device for screening a sample containing phenol, isopropylated phosphate (3:1) according to the present invention made in order to solve the above problems includes:

a measurement value acquisition unit configured to acquire a content of a compound group represented by the following chemical formula 1 (x, y, and z are the number of isopropyl groups each phenyl group has as a substituent) in a sample, or a value corresponding to the content as a measured value, for each of a plurality of compound groups among a compound group in which a total of x, y, and z that three phenyl groups have is 1, a compound group in which the total is 2, and a compound group in which the total is 3; and

[Chemical Formula 1]

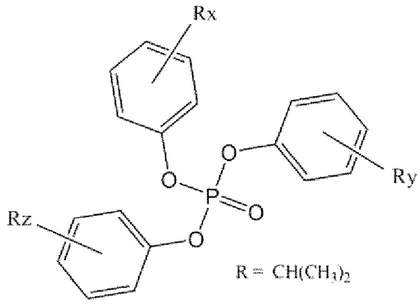

R = $CH(CH_3)_2$

a determination unit configured to determine that the sample contains phenol, isopropylated phosphate (3:1) based on a result of comparing the measured value with a threshold determined previously for the compound group corresponding to the measured value.

ADVANTAGEOUS EFFECTS OF INVENTION

Phenol, isopropylated phosphate (3:1) (PIP (3:1)), which is the compound to be screened in the present invention, is a compound group in which x, y and z in the above chemical formula 1 are all 1 or more. Examples of the compound group represented by the above chemical formula 1 include compounds in which x, y, and z each have a value from 0 to 5. During the production of the product containing PIP (3:1), not only PIP (3:1) but also a compound in which any phenyl group does not have an isopropyl group (R in the above chemical formula) is produced simultaneously as a by-product. In addition, for the product containing PIP (3:1), a compound having a smaller number of substitutions of the isopropyl group is contained more. The present invention has been made based on these findings obtained by the inventors of the present invention.

The present invention acquires, as a measured value, the content or a value corresponding to the content of a plurality of the compound groups, having triphenyl phosphate as the basic structure like PIP (3:1), among a compound group in which the total number of isopropyl groups that three phenyl groups have as substituents is 1, a compound group in which the total number is 2, and a compound group in which the total number is 3. The compound group having a small number of substitutions of isopropyl groups, which is the target compound in the present invention, is contained in a large amount in a sample of a product or the like containing PIP (3:1), and thus a correct measured value can be obtained. Therefore, whether or not the sample to be analyzed contains PIP (3:1) is correctly determined by comparing the measured value of each target compound with a predetermined threshold. The threshold may be a different value or the same value for each compound group. In addition, it is rare that foreign substances having the same mass-to-charge ratio for a plurality of target compounds are simultaneously present in one sample, and thus it is possible to prevent erroneous determination by measuring a plurality of compound groups as in the present invention. Further, screening can be performed more easily than measuring all the compound groups having different numbers of substituents.

BRIEF DESCRIPTION OF DRAWINGS

[Fig. 1] Fig. 1 is a configuration diagram of a main part of a Py/TD-GC/MS device that is an embodiment of a device for screening a sample containing phenol, isopropylated phosphate (3:1) according to the present invention.
[Fig. 2] Fig. 2 is an example of measurement conditions used in the present embodiment.
[Fig. 3] Fig. 3 shows monitoring *m*/*z* and thresholds of compound groups in the present embodiment.
[Fig. 4] Fig. 4 is a calibration curve used in the present embodiment.
[Fig. 5] Fig. 5 is a flowchart showing one embodiment of a method for screening a sample containing phenol, isopropylated phosphate (3:1) according to the present invention.
[Fig. 6] Fig. 6 is a diagram for explaining a determination result in the present embodiment.
[Fig. 7] Fig. 7 is a mass chromatogram obtained by measuring compound groups having different numbers of substituents contained in a resin sample as a standard sample.
[Fig. 8] Fig. 8 is a mass chromatogram obtained by measuring compound groups having different numbers of substituents contained in a resin sample as an industrial product.
[Fig. 9] Fig. 9 is a diagram for explaining a determination method in a first modification.
[Fig. 10] Fig. 10 is a diagram for explaining a determination method in a second modification.

[Fig. 11] Fig. 11 is a diagram for explaining a determination method in a third modification.

DESCRIPTION OF EMBODIMENTS

Embodiments of a method and a device for screening a sample containing phenol, isopropylated phosphate (3:1) (PIP (3:1), CAS: 68937-41-7) according to the present invention will be described below with reference to the drawings.

In the present embodiment, a pyrolyzer/thermal desorption device-gas chromatograph mass spectrometer (Py/TD-GC/MS) 1 is used to screen samples to be analyzed and possibly having PIP (3:1). The sample to be analyzed in the present embodiment is, for example, a resin product, a hydraulic fluid, or an adhesive. PIP (3:1) is defined in Non Patent Literature 1 as a compound group in which x, y, and z are all 1 or more among compounds represented by the following chemical formula 1 (x, y, and z are the number of isopropyl groups each phenyl group has as a substituent).

[Chemical Formula 1]

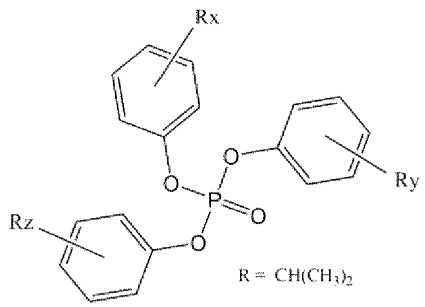

The Py/TD-GC/MS1 roughly includes a gas chromatograph (GC) unit 10, a mass spectrometer (MS) unit 20, and a control/processing unit 30. The gas chromatograph unit 10 includes a sample vaporizing chamber 11, a pyrolyzer (Py) 12, a carrier gas flow path 13 connected to the sample vaporizing chamber 11, and a column 14 connected to an outlet of the sample vaporizing chamber 11. The pyrolyzer 12 can also be used as a thermal desorption device (TD) by changing its heating temperature. The column 14 is accommodated in a column oven 15. Each of the pyrolyzer 12 and the column 14 in the column oven 15 is heated to a predetermined temperature by a heating mechanism (not illustrated).

The mass spectrometer unit 20 includes an electron ionization source 22, an ion lens 23, a quadrupole mass filter 24, and an ion detector 25 in a vacuum chamber 21. Sample components temporally separated in the column 14 are sequentially introduced into the electron ionization source 22, and ionized by irradiation of thermoelectrons emitted from a filament (not illustrated).

The control/processing unit 30 includes a storage unit 31. The storage unit 31 stores a method file in which sample measurement conditions are described. The measurement conditions described in the method file include the temperature of the pyrolyzer 12, the temperature of the column 14, the type and flow rate of the carrier gas, the range of the mass-to-charge ratio to be measured in the scan measurement, the retention time of each compound included in the plurality of compound groups (compound groups 1 to 3), information on the mass-to-charge ratio (monitoring *m*/*z*) of the ion to be measured (target ion) for each compound group, and the like. Figs. 2 and 3 are examples of measurement conditions described in the method file used in Py/TD-GC/MS 1 of the present embodiment (description of the retention time of each compound is omitted). The storage unit 31 also stores information on thresholds (L1 to L3) to be used for comparison with measured values to be described later, and information on a calibration curve to be created based on measurement results of a standard sample to be described later (Fig. 4). In the present embodiment, it may be configured that the presence or absence of PIP (3:1) is determined by setting the values of the thresholds L1 to L3 to the detection lower limit value or a value close thereto.

The control/processing unit 30 includes, as functional blocks, a standard sample measurement unit 32, a calibration curve creation unit 33, an actual sample measurement unit 34, a measurement value acquisition unit 35, a determination unit 36, and an information output unit 37. An entity of the control/processing unit 30 is a general personal computer, and these functional blocks are embodied by executing a screening program installed previously by a processor. An input unit 4 for a user to perform an input operation and a display unit 5 for displaying various types of information are connected to the control/processing unit 30.

Next, a procedure of screening using Py/TD-GC/MS1 of the present embodiment will be described with reference to a flowchart in Fig. 5.

In the present embodiment, a standard sample is measured before screening of an actual sample (step 1). The standard sample used in the present embodiment is a resin sample containing a known amount of a plurality of compound groups represented by the above chemical formula 1 (x, y, and z each represent the number of isopropyl groups that the

phenyl group has as a substituent; a compound group in which the total of x, y, and z is 1 to 3 is included). The measurement of the standard sample only needs to be performed once, and the measurement of the standard sample can be omitted by storing the calibration curve created based on the result in the storage unit 31. In the present embodiment, the resin sample is used as the standard sample, but the type of the standard sample may be appropriately selected according to the type of the sample to be measured (typically, the same type of sample as the sample to be measured is selected).

When a user sets a standard sample in the pyrolyzer 12 and instructs measurement of the standard sample, the standard sample measurement unit 32 reads a method file (refer to Figs. 2 and 3) stored in the storage unit 31 and performs measurement based on contents described in the method file. Specifically, first, the temperature of the pyrolyzer 12 is gradually raised to desorb various compounds including PIP (3:1) contained in the standard sample, and the compounds are introduced into the column 14 on a carrier gas flow. The compound introduced into the column 14 is temporally separated in accordance with the magnitude of the interaction with the liquid phase inside the column 14 and flows out. The compounds flowing out of the column 14 are sequentially introduced into the electron ionization source 22.

The ions generated by the electron ionization source 22 are converged in the vicinity of the central axis (ion optical axis C) in the flight direction by the ion lens 23, then incident on the quadrupole mass filter 24, separated according to the mass-to-charge ratio, and detected by the ion detector 25. The output signals from the ion detector 25 are sequentially transmitted to and stored in the storage unit 31.

During the measurement of the standard sample, the mass spectrometer unit 20 repeatedly performs scan measurement and selected ion monitoring (SIM) measurement. Specifically, a scan measurement in which the mass-to-charge ratio of ions passing through the quadrupole mass filter 24 is scanned in a predetermined range ($m/z$ is 50 to 1000 in the present embodiment) and SIM measurement in which the mass-to-charge ratio of ions passing through the quadrupole mass filter 24 is fixed to the mass-to-charge ratio of target ions of each compound group for a predetermined time are set as one set, and these are repeatedly performed. In the present embodiment, a target ion (ion having monitoring $m/z$) is subjected to SIM measurement for each of a compound group having one substituent (compound group 1), a compound group having two substituents (compound group 2), and a compound group having three substituents (compound group 3). Therefore, in the present embodiment, one scan measurement and three types of SIM measurement constitute one set.

When the measurement of the standard sample is completed, the calibration curve creation unit 33 reads the output signal of the ion detector 25 stored in the storage unit 31. Then, a total ion current chromatogram is created using the output signal obtained at the time of scan measurement. In addition, a mass chromatogram of each of the compound groups 1 to 3 is created using the output signals obtained at the time of the three SIM measurements. The compound groups 1 to 3 include a plurality of compounds having different substitution positions of the isopropyl group, and thus a plurality of mass peaks typically appear in each mass chromatogram. In the present embodiment, a calibration curve of each of the compound groups 1 to 3 is created based on the total of peak areas (or peak heights) of a plurality of mass peaks appearing on the mass chromatogram of one compound group, and the amount of the compound groups 1 to 3 contained in the standard sample (Fig. 4, step 2). Herein, a one-point calibration curve connecting the origin and one measurement point is created, but the calibration curve may be created using the results of measuring a plurality of standard samples having different contents of the compound groups 1 to 3. In addition, herein, the processing of summing the peak areas or peak heights of a plurality of compounds (compounds having different substituent positions) contained in each of the compound groups 1 to 3 is performed, but a calibration curve may be created by selecting one or a plurality of compounds (for example, compounds having high measurement sensitivity) among the plurality of compounds. The calibration curve of the compound groups 1 to 3 created herein is stored in the storage unit 31.

In the present embodiment, information on the retention time of the compound contained in each of the compound groups 1 to 3 is described previously in the method file stored in the storage unit 31 (the retention time is known), but when the retention time is unknown, a retention index may be used. When the retention index is used, the n-alkane sample is measured separately from the measurement of the standard sample. The n-alkane sample is a standard sample containing a plurality of compounds having different lengths of hydrocarbon chains, and is used to obtain a retention index based on the retention time of each compound. The retention index Ix of a compound x is represented by the following formula (1).

$$Ix = 100\ (Cn + i - Cn)\{(tx - tn)/(tn + i - tn)\} + 100\ Cn \quad ...(1)$$

Herein, Cn and Cn+i are the numbers of carbon atoms in the n-alkane whose retention times are located before and after the retention time of the compound, tx is the retention time of the compound x, and tn and tn+i are the retention times of the n-alkane whose retention times are located before and after the retention time of the compound.

Thereafter, when the user inputs information on the sample to be analyzed (sample name and the number of samples), sets the first sample in the pyrolyzer 12, and instructs start of measurement, the actual sample measurement unit 34 reads the method file (refer to Figs. 2 and 3) stored in the storage unit 31, and executes measurement based on the content described in the method file, similarly to the standard sample measurement unit 32 (step 3). Similarly to the

measurement of the standard sample, the output signal from the ion detector 25 is sequentially sent to the storage unit 31 and stored.

When the measurement of the actual sample is completed, the measurement value acquisition unit 35 reads the output signal of the ion detector 25 stored in the storage unit 31, and creates a total ion current chromatogram and a mass chromatogram corresponding to each of the compound groups 1 to 3, similarly to the measurement of the standard sample. Then, the area (or peak height) of a mass peak of each compound belonging to the compound group on the mass chromatogram of each of the compound groups 1 to 3 is determined, and the sum thereof is calculated. Then, the content of each of the compound groups 1 to 3 is obtained based on the calibration curve stored in the storage unit 31 (step 4).

When the content of the compound groups 1 to 3 is acquired, the determination unit 36 calculates the concentration of each of the compound groups 1 to 3 from the weight of the sample to be analyzed (the weight of the sample set in the pyrolyzer 12) and the content. Subsequently, the thresholds L1 to L3 associated with the respective compound groups 1 to 3 are read and compared (step 5, refer to Fig. 6). Then, it is determined whether or not the concentrations of a plurality of compound groups in the compound groups 1 to 3 exceed a threshold (step 6).

When the number of compound groups having a concentration exceeding the threshold is 0 or 1 (NO in step 6), the determination unit 36 determines that there is a high possibility that the sample does not contain PIP (3:1). Meanwhile, when the concentrations of the plurality of compound groups exceed the threshold (YES in step 6, refer to Fig. 6), the determination unit 36 determines that there is a high possibility that PIP (3:1) is contained in the sample. When it is determined that there is a high possibility that PIP (3:1) is contained, the information output unit 37 displays, on the display unit 5, that there is a high possibility that PIP (3:1) is contained in the sample (step 7). In addition, the number of measured compound groups and the number of compound groups whose concentrations exceed the threshold are simultaneously displayed (for example, (number of compound groups whose concentration ≥ threshold)/number of compound groups to be measured (2/3, 3/3, or the like) is displayed). This facilitates the understanding of the level of possibility that PIP (3:1) is contained. Alternatively, when the concentrations of all the compound groups exceed the threshold, it may be displayed in a color (for example, red) in which it can be seen that PIP (3:1) is contained, and when the concentrations of some of the compound groups exceed the threshold, it may be displayed in a color (for example, yellow) in which it can be seen that there is a possibility that PIP (3:1) is contained, thereby prompting the user to perform more detailed analysis.

When the above processing by the determination unit 36 and the information output unit 37 is completed, the actual sample measurement unit 34 confirms the presence or absence of an unmeasured sample, and when there is an unmeasured sample (YES in step 8), the process returns to step 3 and repeats the same processing as described above. When there is no unmeasured sample (NO in step 8), the series of processing is ended.

As described above, in the present embodiment, in order to determine whether or not the sample to be analyzed contains PIP (3:1), a group of compounds having a basic structure common to PIP (3:1) and having the number of substitutions of the isopropyl group of 1 to 3 is measured. This is based on the fact that, as a result of measuring various samples containing PIP (3:1), the inventors of the present invention have found that samples containing PIP (3:1) contain not only PIP (3:1) but also compounds in which any phenyl group does not have an isopropyl group R as a by-product, and in many cases, their contents are higher than PIP (3:1).

Fig. 7 shows a total ion current chromatogram (TIC) obtained by measuring a sample (corresponding to a resin weight of 0.5 mg) obtained by adding a standard solution prepared from PIP (3:1) sold as a standard reagent and a PVC solution to a sample cup for Py/TD-GC/MS analysis and setting the concentration of PIP (3:1) in the resin to 1%, and a mass chromatogram of compound groups 0 to 6 having different numbers of substituents. In addition, Fig. 8 shows a TIC obtained by measuring a sample (corresponding to a resin weight of 0.5 mg) obtained by adding a solution prepared from PIP (3:1) sold as an industrial product and a PVC solution to a sample cup for Py/TD-GC/MS analysis and setting the concentration of PIP (3:1) in the resin to 1%, and a mass chromatogram of compound groups 0 to 6 having different numbers of substituents. The title iPr:x in the figure represents a compound group in which the number of substitutions of the isopropyl group is x. In addition, the numerical value of *m*/*z* represents the mass-to-charge ratio (monitoring *m*/*z*) of the target ion of the compound group, and the value in parentheses represents the magnification ratio of the chromatogram. These are an example of measurement, but it can be seen that samples having the number of substitutions of 0 to 3 are detected in any sample.

In addition, as a result of mass spectrometry of a compound group having different numbers of substituents, it has been also found that the measurement sensitivity tends to be higher as the number of substituents is smaller. Therefore, as in the present embodiment, using the concentration of the compound groups 1 to 3, which is contained in a large amount in a sample containing PIP (3:1) and has high measurement sensitivity, as a criterion of determination can completely screen a sample containing PIP (3:1).

During the production of the product containing PIP (3:1), not only PIP (3:1) but also a compound in which any phenyl group does not have an isopropyl group (R in the above chemical formula 1) is produced simultaneously as a by-product. It is not easy to selectively remove such by-product, or selectively extract only PIP (3:1), or produce only PIP (3:1) so that no by-products are generated, and thus products containing PIP (3:1) contain such by-products with a high probability. Therefore, it can be said that instead of directly measuring PIP (3:1), determining that PIP (3:1) is contained

based on the fact that the compound groups 0 to 3 generated as by-products of PIP (3:1) are contained is appropriate as a determination method.

In the above embodiment, the concentration of the compound groups 1 to 3 is compared with the threshold, but only two of the compound groups 1 to 3 may be measured. In a case where there is a foreign substance that generates an ion having the monitoring *m*/*z* set for the compound group, measurement of only one of the compound groups arises a possibility that it is erroneously determined that the compound group is measured although the foreign substance is detected, and determination of screening becomes erroneous. Therefore, it is preferable to compare the concentrations of the plurality of compound groups with the threshold. In addition, in the above embodiment, the concentration of each of the compound groups is obtained and compared with the threshold, but if the weight of the sample to be measured is constant, the threshold of the content may be set.

The above-described embodiment is merely an example. Some modifications will be described below.

<First Modification>

In the above embodiment, the concentrations of a plurality of compound groups of the compound groups 1 to 3 in the sample to be analyzed were compared with the threshold, but as shown in Figs. 7 and 8, the sample containing PIP (3:1) also contains triphenyl phosphate (the number of substituents is 0) and many compounds having four isopropyl groups, and these can also be measured with high sensitivity. Therefore, the target may be expanded to the compound groups 0 to 4 (the number of substituents is 0 to 4), and the concentrations of a plurality of compound groups among them may be compared with the threshold. Fig. 9 shows an example in which the concentration of the compound groups 0 to 4 was compared with the threshold (example in which the concentration of the compound groups 0 to 3 is equal to or more than the threshold and only the concentration of the compound group 4 is less than the threshold). As described above, by expanding the compound groups to be measured, determination based on the measured values of more compound groups can be performed, and thus the accuracy of the determination is further improved.

<Second Modification>

In the above embodiment, a threshold is set for each of the compound groups 1 to 3, and the sample is screened based on the result of comparing the concentration of the compound groups 1 to 3 contained in the sample to be analyzed with each threshold. However, in addition to this, a threshold (total threshold) may be set for the total concentration of the compound groups (for example, the compound groups 1 to 3) to be measured, and the sample may be screened in consideration of the result of comparing the total concentration of a plurality of compound groups obtained by measurement with the threshold. Fig. 10 shows an example in which the total concentration of the compound groups 1 to 3 was compared with the total threshold (an example in which the total concentration of the compound groups 1 to 3 does not exceed the total threshold).

In PIP (3:1), the ratio of the content of the compound groups for each number of substitutions of the isopropyl group may vary depending on the production conditions and the like, and thus the concentration of a specific compound group may be low. In addition, ionization of a specific compound group is suppressed (ion suppression) due to the influence of other components contained in the sample, and the measurement sensitivity may be lowered. Even in these cases, in the second modification, the possibility of missing presence of PIP (3:1) in the sample can be reduced by comparing the sum with the measured values of other compound groups with the threshold.

<Third Modification>

In the above embodiment, the content of each of the compound groups is determined from the peak area (or peak height) of the mass chromatogram, and the concentration of each of the compound groups is determined based on the content and the weight of the sample. However, as in the above embodiment, when the device for measuring a standard sample and the device for measuring a sample to be analyzed are the same or of the same type and the measurement conditions are substantially the same, the peak area or the peak height may be compared with the threshold. Fig. 11 shows an example in which the peak height was compared with the threshold for each of the compound groups 1 to 3 (an example in which all the peak heights of the compound groups 1 to 3 are equal to or more than the threshold). As a result, it is not necessary to perform a process of determining the content by collating the peak area and the peak height obtained by the measurement with the calibration curve, and the determination can be performed more easily.

PIP (3:1) is a collection of many compounds, and thus the content and concentration of each of the compounds may not be accurately known even for a standard sample. In the third modification, even in such a case, using the measurement result of the standard sample or the like as a reference and using its peak area or peak height as a threshold can provide screen determination based on whether or not the compound group to be measured is contained at a higher concentration than the standard sample.

In the above embodiment and modifications, the configuration in which a sample containing PIP (3:1) is screened by measurement using Py/TD-GC/MS has been described. However, other measurement methods can be used as long as the compound group can be measured as in the above embodiment and modifications. For example, it is possible to adopt a configuration in which chromatography (gas chromatography, liquid chromatography, or the like that detects each compound by spectroscopic measurement or the like) using only a chromatograph is performed, and each compound separated in the column is measured. However, using the mass spectrometry as described above can measure a plurality of compounds having the same number of substitutions by one common monitoring *m*/*z*, and thus it is preferable to use the mass spectrometry. In addition, when Py/TD is used, pretreatment such as dissolving a sample in a solvent is unnecessary, and thus Py/TD is preferably used. In addition, mass spectrometry (TD-MS) or the like not using chromatographic separation may be used.

[Modes]

It is understood by those skilled in the art that the plurality of exemplary embodiments described above are specific examples. The scope of the invention is defined by the following claims.

REFERENCE SIGNS LIST

| | |
|---|---|
| 1 | Pyrolyzer/Thermal Desorption-Gas Chromatograph Mass Spectrometer |
| 10 | Gas Chromatograph Unit |
| 11 | Sample Vaporizing Chamber |
| 12 | Pyrolyzer |
| 13 | Carrier Gas Flow Path |
| 14 | Column |
| 15 | Column Oven |
| 20 | Mass Spectrometry Unit |
| 21 | Vacuum Chamber |
| 22 | Electron Ionization Source |
| 23 | Ion Lens |
| 24 | Quadrupole Mass Filter |
| 25 | Ion Detector |
| 30 | Control/Processing Unit |
| 31 | Storage Unit |
| 32 | Standard Sample Measurement Unit |
| 33 | Calibration Curve Creation Unit |
| 34 | Actual Sample Measurement Unit |
| 35 | Measurement Value Acquisition Unit |
| 36 | Determination Unit |
| 37 | Information Output Unit |
| 4 | Input Unit |
| 5 | Display Unit |

## Claims

**1.** A method for screening a sample containing phenol, isopropylated phosphate (3:1) comprising:

a step of acquiring a content of a compound group represented by the following chemical formula 1 (x, y, and z are the number of isopropyl groups each phenyl group has as a substituent) in a sample, or a value corresponding to the content as a measured value, for each of a plurality of compound groups among a compound group in which a total of x, y, and z that three phenyl groups have is 1, a compound group in which the total is 2, and a compound group in which the total is 3;

[Chemical Formula 1]

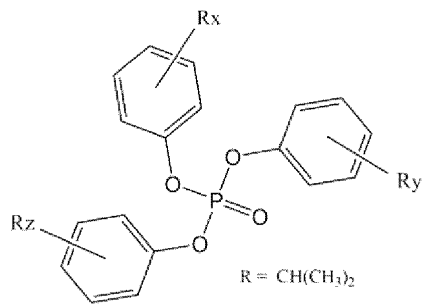

a step of comparing the measured value with a threshold determined previously for the compound group corresponding to the measured value; and
a step of determining that the sample contains phenol, isopropylated phosphate (3:1) based on the comparison result.

**2.** The method for screening a sample containing phenol, isopropylated phosphate (3:1) according to claim 1, wherein, in the step of acquiring the measured value, a content of triphenyl phosphate and/or a compound group represented by the above chemical formula 1 in which the total of x, y, and z is 4 or more in the sample, or a value corresponding to the content is further acquired as a measured value.

**3.** The method for screening a sample containing phenol, isopropylated phosphate (3:1) according to claim 1, wherein, in the step of comparing with the threshold, a total of the measured values of the plurality of compound groups is compared with a predetermined threshold.

**4.** The method for screening a sample containing phenol, isopropylated phosphate (3:1) according to claim 1, wherein, in the step of acquiring the measured value, data of a measurement peak corresponding to each of the plurality of compound groups is acquired, and the measured value that is a height or an area of the measurement peak is acquired as the value corresponding to the content.

**5.** The method for screening a sample containing phenol, isopropylated phosphate (3:1) according to claim 1, wherein information prompting confirmation of the sample is output when only some of the measured values of the plurality of compound groups exceed the predetermined threshold.

**6.** A device for screening a sample containing phenol, isopropylated phosphate (3:1) comprising:

a measurement value acquisition unit (35) configured to acquire a content of a compound group represented by the following chemical formula 1 (x, y, and z are the number of isopropyl groups each phenyl group has as a substituent) in a sample, or a value corresponding to the content as a measured value, for each of a plurality of compound groups among a compound group in which a total of x, y, and z that three phenyl groups have is 1, a compound group in which the total is 2, and a compound group in which the total is 3;

[Chemical Formula 1]

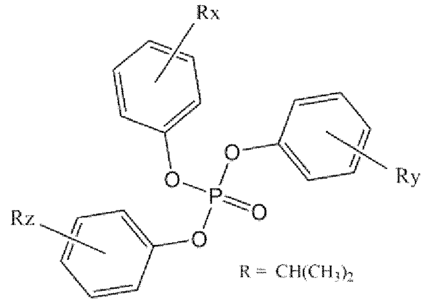

a measurement value comparison unit configured to compare the measured value with a threshold determined

previously for the compound group corresponding to the measured value; and
a determination unit (36) configured to determine that the sample contains phenol, isopropylated phosphate (3:1) based on a comparison result of the measurement value comparison unit.

## Patentansprüche

1. Verfahren zum Screening einer Probe, die Phenol, isopropyliertes Phosphat (3:1) enthält, umfassend:

   einen Schritt des Erfassens eines Gehalts einer Verbindungsklassengruppe, dargestellt durch die nachstehende chemische Formel 1 (wobei x, y und z die Anzahl der Isopropylgruppen sind, die jede Phenylgruppe als Substituenten aufweist), in einer Probe, oder eines dem Gehalt entsprechenden Wertes als Messwert, für jede von mehreren Verbindungsklassengruppen unter einer Verbindungsklassengruppe, bei der die Summe von x, y und z, die drei Phenylgruppen aufweisen, 1 beträgt, einer Verbindungsklassengruppe, bei der die Summe 2 beträgt, und einer Verbindungsklassengruppe, bei der die Summe 3 beträgt;

   [Chemische Formel 1]

   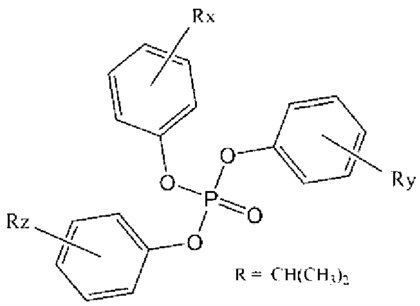

   einen Schritt des Vergleichens des Messwerts mit einem zuvor festgelegten Schwellenwert für die Verbindungsklassengruppe, die dem Messwert entspricht; und
   einen Schritt des Bestimmens, dass die Probe Phenol, isopropyliertes Phosphat (3:1) enthält, basierend auf dem Vergleichsergebnis.

2. Verfahren zum Screening einer Probe, die Phenol, isopropyliertes Phosphat (3:1) enthält, nach Anspruch 1, wobei in dem Schritt des Erfassens des Messwerts zusätzlich ein Gehalt an Triphenylphosphat und/oder einer durch die vorstehende chemische Formel 1 dargestellten Verbindungsklassengruppe, bei der die Summe von x, y und z 4 oder mehr beträgt, in der Probe, oder ein dem Gehalt entsprechender Wert, als Messwert erfasst wird.

3. Verfahren zum Screening einer Probe, die Phenol, isopropyliertes Phosphat (3:1) enthält, nach Anspruch 1, wobei in dem Schritt des Vergleichens mit dem Schwellenwert eine Summe der Messwerte der mehreren Verbindungsklassengruppen mit einem zuvor festgelegten Schwellenwert verglichen wird.

4. Verfahren zum Screening einer Probe, die Phenol, isopropyliertes Phosphat (3:1) enthält, nach Anspruch 1, wobei in dem Schritt des Erfassens des Messwerts Daten eines Messpeaks, der jeder der mehreren Verbindungsklassengruppen entspricht, erfasst werden, und der Messwert, der eine Höhe oder eine Fläche des Messpeaks ist, als der dem Gehalt entsprechende Wert erfasst wird.

5. Verfahren zum Screening einer Probe, die Phenol, isopropyliertes Phosphat (3:1) enthält, nach Anspruch 1, wobei Informationen, die zur Bestätigung der Probe auffordern, ausgegeben werden, wenn nur einige der Messwerte der mehreren Verbindungsklassengruppen den zuvor festgelegten Schwellenwert überschreiten.

6. Vorrichtung zum Screening einer Probe, die Phenol, isopropyliertes Phosphat (3:1) enthält, umfassend:

   eine Messwerterfassungseinheit (35), die dazu ausgebildet ist, einen Gehalt einer durch die nachstehende chemische Formel 1 dargestellten Verbindungsklassengruppe (wobei x, y und z die Anzahl der Isopropylgruppen sind, die jede Phenylgruppe als Substituenten aufweist) in einer Probe, oder einen dem Gehalt entsprechenden Wert als Messwert, für jede von mehreren Verbindungsklassengruppen unter einer Verbindungsklassengruppe,

bei der die Summe von x, y und z, die drei Phenylgruppen aufweisen, 1 beträgt, einer Verbindungsklassengruppe, bei der die Summe 2 beträgt, und einer Verbindungsklassengruppe, bei der die Summe 3 beträgt, zu erfassen;

[Chemische Formel 1]

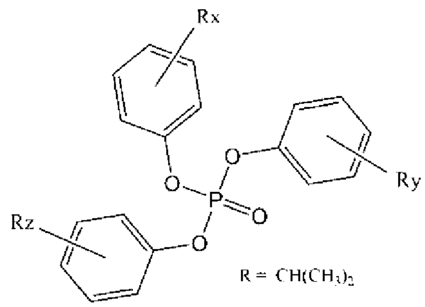

eine Messwertvergleichseinheit, die dazu ausgebildet ist, den Messwert mit einem zuvor festgelegten Schwellenwert für die dem Messwert entsprechende Verbindungsklassengruppe zu vergleichen; und
eine Bestimmungseinheit (36), die dazu ausgebildet ist, zu bestimmen, dass die Probe Phenol, isopropyliertes Phosphat (3:1) enthält, basierend auf einem Vergleichsergebnis der Messwertvergleichseinheit.

**Revendications**

**1.** Procédé de criblage d'un échantillon contenant du phosphate isopropylé phénol (3/1) comprenant :

une étape d'acquisition d'une teneur en un groupe de composé représenté par la formule chimique suivante 1 (x, y et z sont le nombre de groupes isopropyle que chaque groupe phényle a en tant que substituant) dans un échantillon, ou d'une valeur correspondant à la teneur en tant que valeur mesurée, pour chacun d'une pluralité de groupes de composé parmi un groupe de composé dans lequel un total de x, y et z que trois groupes phényle ont est de 1, un groupe de composé dans lequel le total est de 2 et un groupe de composé dans lequel le total est de 3 ;

[Formule chimique 1]

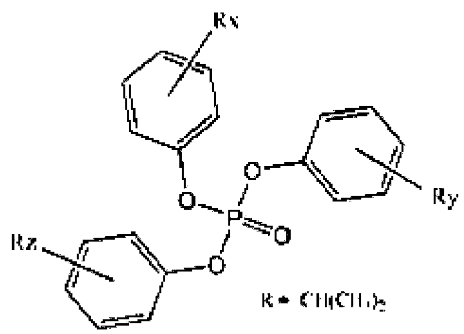

une étape de comparaison de la valeur mesurée à un seuil déterminé précédemment pour le groupe de composé correspondant à la valeur mesurée ; et
une étape de détermination que l'échantillon contient du phosphate isopropylé phénol (3/1) en fonction du résultat de comparaison.

**2.** Procédé de criblage d'un échantillon contenant du phosphate isopropylé phénol (3/1) selon la revendication 1, dans lequel, dans l'étape d'acquisition de la valeur mesurée, une teneur en phosphate triphénylique et/ou un groupe de composé représenté par la formule chimique ci-dessus 1 dans laquelle le total de x, y et z est de 4 ou plus dans l'échantillon, ou une valeur correspondant à la teneur est en outre acquise en tant que valeur mesurée.

**3.** Procédé de criblage d'un échantillon contenant du phosphate isopropylé phénol (3/1) selon la revendication 1, dans lequel, dans l'étape de comparaison au seuil, un total des valeurs mesurées de la pluralité de groupes de composé est

comparé à un seuil prédéterminé.

**4.** Procédé de criblage d'un échantillon contenant du phosphate isopropylé phénol (3/1) selon la revendication 1, dans lequel, dans l'étape d'acquisition de la valeur mesurée, des données d'un pic de mesure correspondant à chacun de la pluralité de groupes de composé sont acquises, et la valeur mesurée qui est une hauteur ou une surface du pic de mesure est acquise en tant que valeur correspondant à la teneur.

**5.** Procédé de criblage d'un échantillon contenant du phosphate isopropylé phénol (3/1) selon la revendication 1, dans lequel des informations invitant à la confirmation de l'échantillon sont sorties lorsque seules certaines des valeurs mesurées de la pluralité de groupes de composé dépassent le seuil prédéterminé.

**6.** Dispositif de criblage d'un échantillon contenant du phosphate isopropylé phénol (3/1) comprenant :

un module d'acquisition de valeur de mesure (35) configuré pour acquérir une teneur en un groupe de composé représenté par la formule chimique suivante 1 (x, y et z sont le nombre de groupes isopropyle que chaque groupe phényle a en tant que substituant) dans un échantillon, ou une valeur correspondant à la teneur en tant que valeur mesurée, pour chacun d'une pluralité de groupes de composé parmi un groupe de composé dans lequel un total de x, y et z que trois groupes phényle ont est de 1, un groupe de composé dans lequel le total est de 2 et un groupe de composé dans lequel le total est de 3 ;

[Formule chimique 1]

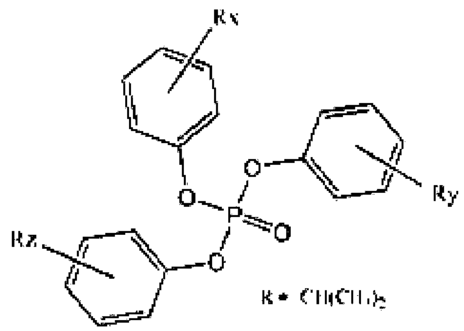

un module de comparaison de valeur de mesure configuré pour comparer la valeur mesurée à un seuil déterminé précédemment pour le groupe de composé correspondant à la valeur mesurée ; et
un module de détermination (36) configuré pour déterminer que l'échantillon contient du phosphate isopropylé phénol (3/1) en fonction d'un résultat de comparaison du module de comparaison de valeur de mesure.

Fig. 1

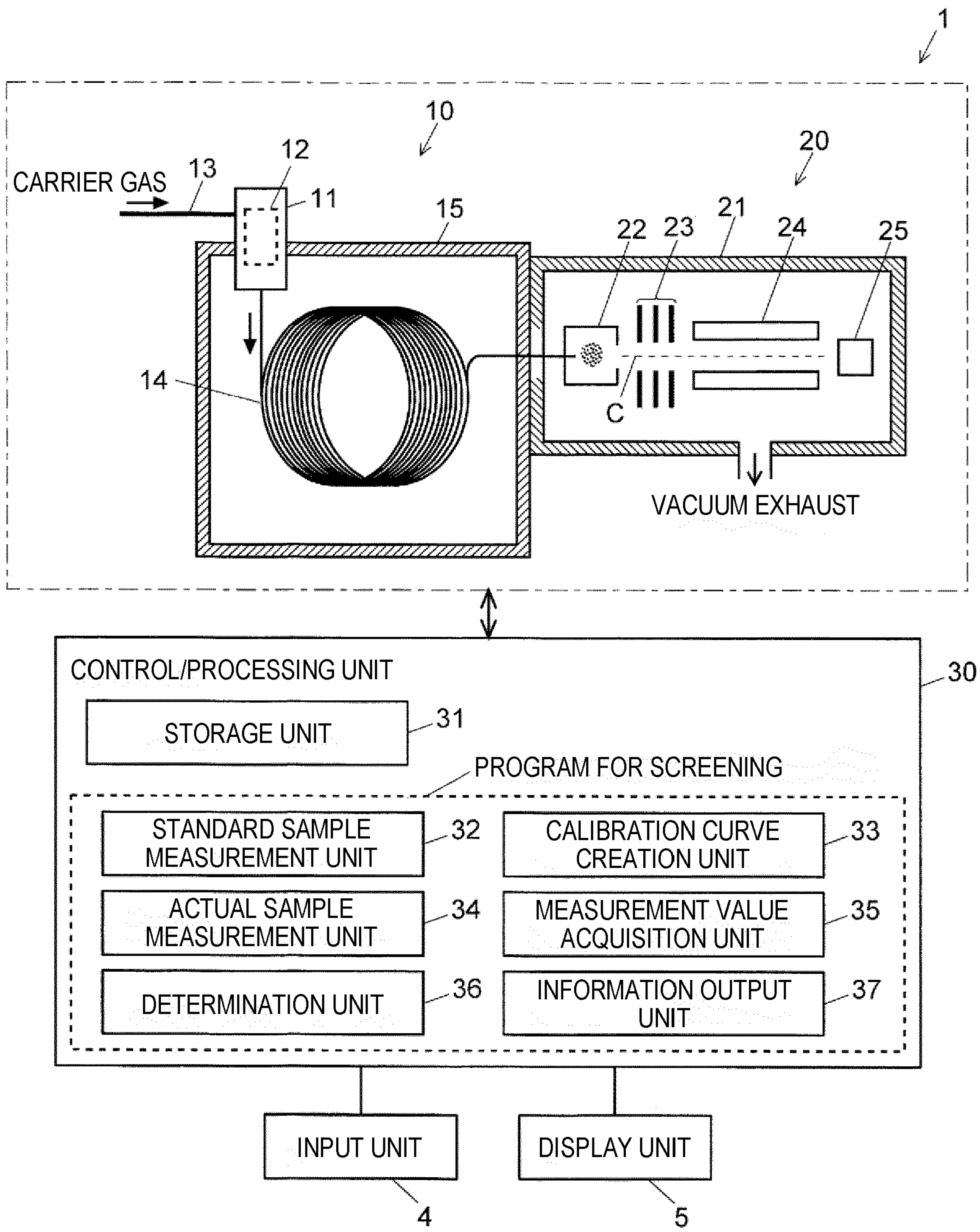
1
10
20
CARRIER GAS
13
12
11
15
22
23
21
24
25
14
C
VACUUM EXHAUST
CONTROL/PROCESSING UNIT
30
STORAGE UNIT
31
PROGRAM FOR SCREENING
STANDARD SAMPLE MEASUREMENT UNIT
32
CALIBRATION CURVE CREATION UNIT
33
ACTUAL SAMPLE MEASUREMENT UNIT
34
MEASUREMENT VALUE ACQUISITION UNIT
35
DETERMINATION UNIT
36
INFORMATION OUTPUT UNIT
37
INPUT UNIT
DISPLAY UNIT
4
5

# Fig. 2

PYROLYZER

| | |
|---|---|
| HEATING TEMPERATURE: | 200 °C→ (20 °C/min) →300 °C→ (5 °C/min) →340 °C (1 min) |
| INTERFACE TEMPERATURE: | 300 °C |

GC

| | |
|---|---|
| INJECTION TEMPERATURE: | 300 °C |
| CONTROL MODE: | LINEAR VELOCITY CONTROL (52.1 cm/sec) |
| INJECTION MODE: | SPLIT (SPLIT RATIO 50) |
| PURGE FLOW RATE: | 3.0 ml/min |
| CARRIER GAS | He |
| COLUMN: | SH-IMS (15 m, 0.25 mm I.D., 0.1 µm + 2m GUARD COLUMN) (SHIMAZU CORPORATION) |
| GC OPEN PROGRAM: | 80 °C→ (20 °C/min) →320 °C (4 min) |

MS

| | |
|---|---|
| INTERFACE TEMPERATURE: | 320 °C |
| ION SOURCE TEMPERATURE: | 230 °C |
| IONIZATION MODE: | EI METHOD |
| MEASUREMENT MODE: | SCAN/SIM SIMULTANEOUS MEASUREMENT |
| SCAN RANGE: | 50 - 1,000 amu |

Py/TD-GC/MS ANALYSIS CONDITION

# Fig. 3

| ID | COMPOUND | MONITORING *m/z* | CONCENTRATION THRESHOLD |
|---|---|---|---|
| 1 | Isopropyl (iPr:1) Trisphenyl phosphate | M1 | L1 |
| 2 | Isopropyl (iPr:2) Trisphenyl phosphate | M2 | L2 |
| 3 | Isopropyl (iPr:3) Trisphenyl phosphate | M3 | L3 |

# Fig. 4

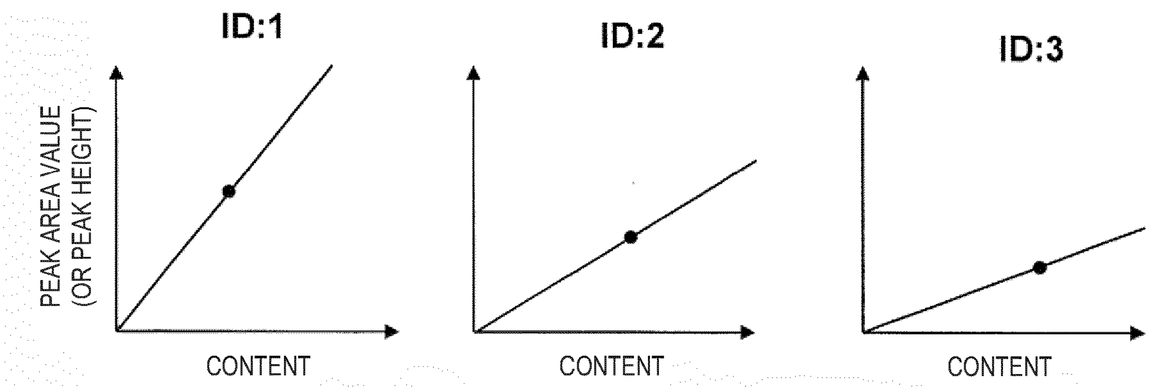

Fig. 5

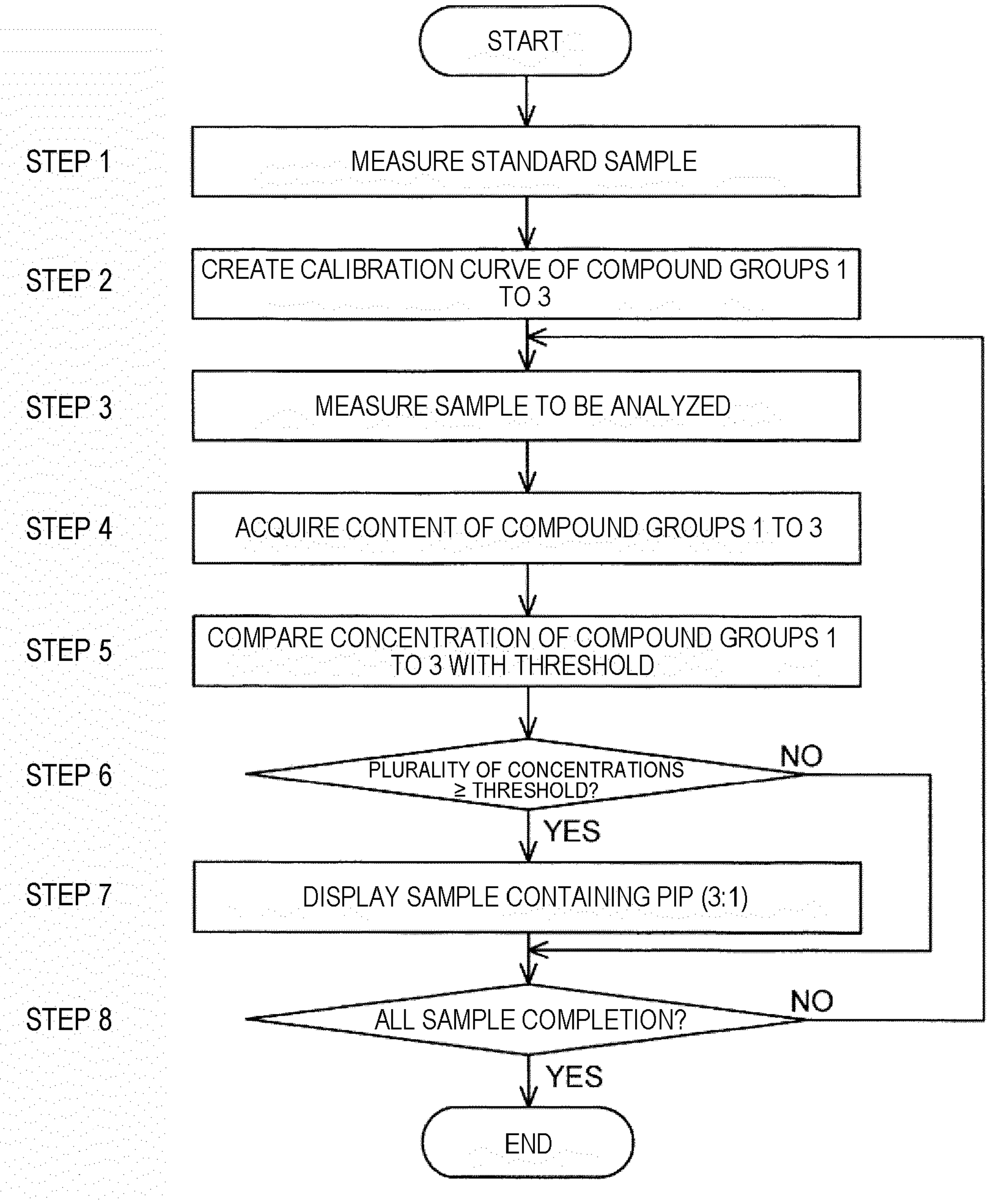


Fig. 6

| ID | COMPOUND | A: QUANTITATIVE CONCENTRATION VALUE | B CONCENTRATION THRESHOLD | A≥B ? | DETERMINATION OF PIP (3:1) CONTAINED (CONDITION: TWO OR MORE OF YES) |
|---|---|---|---|---|---|
| 1 | Isopropyl (iPr:1) Trisphenyl phosphate | C1 | L1 | Yes | CONTAINED |
| 2 | Isopropyl (iPr:2) Trisphenyl phosphate | C2 | L2 | Yes | |
| 3 | Isopropyl (iPr:3) Trisphenyl phosphate | C3 | L3 | Yes | |

Fig. 7

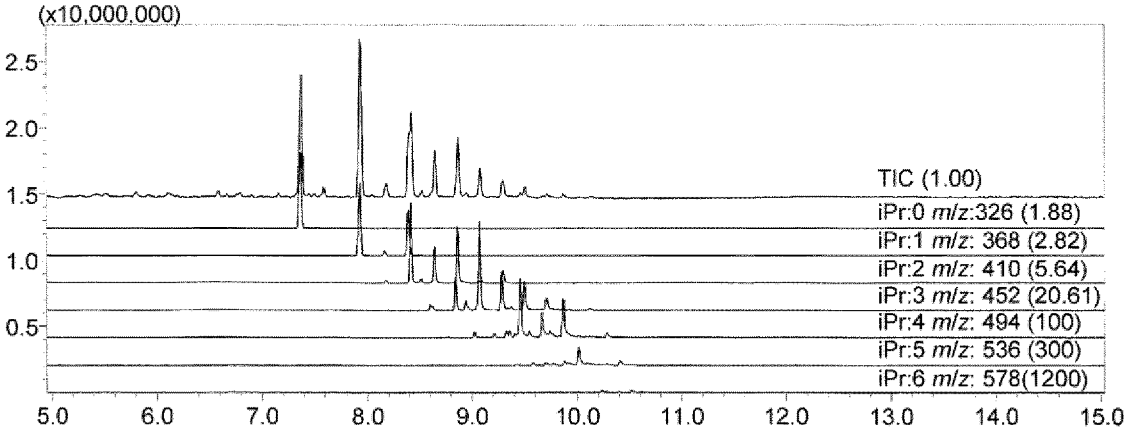


Fig. 8

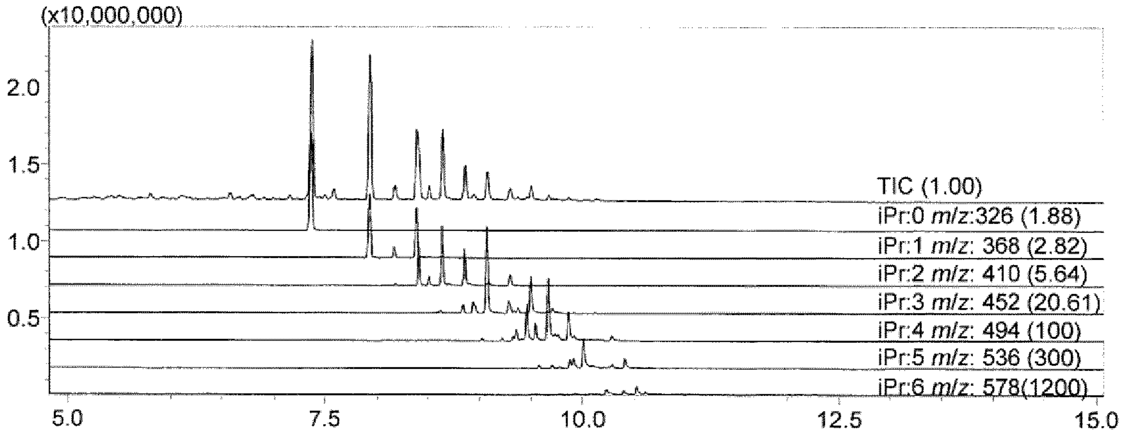


Fig. 9

| ID | COMPOUND | A: QUANTITATIVE CONCENTRATION VALUE | B CONCENTRATION THRESHOLD | C: A≧B ? | DETERMINATION OF PIP (3:1) CONTAINED (CONDITION: 4 OR MORE OF YES) |
|---|---|---|---|---|---|
| 1 | Isopropyl (iPr:1) Trisphenyl phosphate | C1 | L1 | Yes | CONTAINED |
| 2 | Isopropyl (iPr:2) Trisphenyl phosphate | C2 | L2 | Yes | |
| 3 | Isopropyl (iPr:3) Trisphenyl phosphate | C3 | L3 | Yes | |
| 4 | Isopropyl (iPr:0) Trisphenyl phosphate | C0 | L0 | Yes | |
| 5 | Isopropyl (iPr:4) Trisphenyl phosphate | C4 | L4 | No | |

Fig. 10

| ID | COMPOUND | A: QUANTITATIVE CONCENTRATION VALUE | B CONCENTRATION THRESHOLD | C1+C2+C3 ≥B ? | DETERMINATION OF PIP (3:1) CONTAINED (CONDITION: YES) |
|---|---|---|---|---|---|
| 1 | Isopropyl (iPr:1) Trisphenyl phosphate | C1 | La | No | NOT CONTAINED |
| 2 | Isopropyl (iPr:2) Trisphenyl phosphate | C2 | | | |
| 3 | Isopropyl (iPr:3) Trisphenyl phosphate | C3 | | | |

Fig. 11

| ID | COMPOUND | A: PEAK HEIGHT | B PEAK HEIGHT THRESHOLD | A≥B ? | DETERMINATION OF PIP (3:1) CONTAINED (CONDITION: THREE OF YES) |
|---|---|---|---|---|---|
| 1 | Isopropyl (iPr:1) Trisphenyl phosphate | H1 | L1 | Yes | CONTAINED |
| 2 | Isopropyl (iPr:2) Trisphenyl phosphate | H2 | L2 | Yes | |
| 3 | Isopropyl (iPr:3) Trisphenyl phosphate | H3 | L3 | Yes | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS*, 68937-41-7
- Preliminary Information on Manufacturing, Processing, Distribution, Use, and Disposal: Phenol, isopropylated, phosphate (3:1). *Support document for Docket EPA-HQ-OPPT-2016-073*, 2017
- EPA Finalizes Action Protecting Americans from PBT Chemicals. United States Environmental Protection Agency, December 22, 2020. 20 August 2021
- **MARTIN SCHLUMMERA et al.** Analysis of flame retardant additives in polymer fractions of waste of electric and electronic equipment (WEEE) by means of HPLC-UV/MS and GPC-HPLC-UV. *Journal of Chromatography A*, 28 January 2005, vol. 1064 (1), 39-51
- **THOMAS ROTH et al.** *Gas chromatographic determination of phosphate-based flame retardants in styrene-based polymers from waste electrical and electronic equipment*, 02 November 2012, vol. 1262, 188-195
- **TOSHINARI ISHII** ; **YUKIHIKO KUDO** ; **KENICHI OBAYASHI**. Application news pyrolysis (thermal desorption) - Analysis of PIP (3:1) in resin using GC/MS. Shimadzu Corporation, June 2021
- **ROSENBERGER W et al.** Triphenyl phosphate, isopropylated (isopropylated phenyl phosphates, IPPhP) -Determination of isopropylated phenyl phosphates in the workplace air using gas chromatography (GC-MS) Air Monitoring Method -Translation of the German version from 2020. *THE MAK COLLECTION FOR OCCUPATIONAL HEALTH AND SAFETY 2021*, March 2021, vol. 6 (1)